# EUROPÄISCHE PATENTSCHRIFT

(11) **EP 1 784 644 B1**
(45) Veröffentlichungstag und Bekanntmachung des Hinweises auf die Patenterteilung: **22.10.2008**
(21) Anmeldenummer: 05778676.6
(22) Anmeldetag: 22.08.2005
(51) Int. Cl.: G01N 33/543, G01N 35/00, B03C 1/021

(54) **MIKROFLUIDISCHES SYSTEM ZUR ISOLIERUNG BIOLOGISCHER PARTIKEL UNTER VERWENDUNG DER IMMUNOMAGNETISCHEN SEPARATION**
MICROFLUID SYSTEM FOR THE ISOLATION OF BIOLOGICAL PARTICLES USING IMMUNOMAGNETIC SEPARATION
SYSTEME MICROFLUIDIQUE SERVANT A ISOLER DES PARTICULES BIOLOGIQUES PAR SEPARATION IMMUNOMAGNETIQUE

(30) Priorität: 23.08.2004 DE 102004040785
(43) Veröffentlichungstag der Anmeldung: 16.05.2007
(73) Patentinhaber: Kist-Europe Forschungsgesellschaft Mbh, 66123 Saarbrücken (DE)
(72) Erfinder: KIM, Jungtae, 66125 Dudweiler (DE); STEINFELD, Ute, 66386 St. Ingbert (DE); SCHUHMACHER, Jörg, 66386 St. Ingbert (DE)
(74) Vertreter: Pfenning, Meinig & Partner GbR
(86) Internationale Anmeldenummer: PCT/EP2005/009065
(87) Internationale Veröffentlichungsnummer: WO 2006/021410

(56) Entgegenhaltungen:
- WO-A-01/24850
- WO-A-02/26292
- US-A- 5 972 721
- US-A1- 2003 044 832

## Beschreibung

Die vorliegende Erfindung bezieht sich auf eine Vorrichtung und ein Verfahren zur Isolation biologischer Partikel. Unter biologischen Partikeln (im folgenden auch alternativ als biologische Materialien bezeichnet) werden Partikel bzw. Materialien auf partikulärer oder molekularer Basis verstanden. Hierzu gehören Zellen, wie beispielsweise Viren oder Bakterien, insbesondere jedoch auch humane und tierische isolierte Zellen, wie Leukozyten oder Tumorzellen, sowie nieder- und hochmolekulare chemische Verbindungen, wie Proteine und Moleküle, insbesondere immunologisch aktive Verbindungen wie Antigene, Antikörper und Nukleinsäuren oder auch antigenspezifische Tetramere, wie beispielsweise MHC-Tetramere oder auch Streptamere. Die vorliegende Erfindung bezieht sich insbesondere auf immunomagnetische Separationstechniken (IMS) für menschliche oder tierische Zellen, automatische Probenpräparationstechniken sowie (elektro)magnetische bzw. magnetische Separationstechniken (EMS) und mikrofluidische Techniken. Die immunomagnetischen Separationstechniken werden unter Einsatz von immunomagnetischen Partikeln durchgeführt. Unter immunomagnetischen Partikeln werden magnetisierbare oder magnetische, beispielsweise ferromagnetische oder superparamagnetische Partikel oder auch weichmagnetische Materialien, wie beispielsweise Ferrite, verstanden, welche so gekennzeichnet sind (beispielsweise durch Kopplung mit einem Antikörper oder einem antigenspezifischen Tetramer), dass sie zur spezifischen Bindung an ein bestimmtes biologisches Material bzw. an einen bestimmten biologischen Partikel fähig sind. Die zur Bindung befähigten, immunomagnetischen Partikel haben bevorzugt im wesentlichen Kugelform (und werden daher alternativ im folgenden auch als immunomagnetische Kügelchen bzw. Antikörpergekoppelte, magnetische Kügelchen bezeichnet) und weisen bevorzugt Korngrößen von weniger als 100 µm auf.

Aufgrund der unterschiedlichen Immuncharakteristiken von biologischen Partikeln können bestimmte Partikel (beispielsweise Antigene oder antigenspezifische Tetramere oder Streptamere) durch bestimmte Antikörper gekennzeichnet werden bzw. an bestimmte Antikörper gebunden werden (Immunreaktion bzw. Antigen-Antikörper-Reaktion).

Als Tetramere werden in der Immunologie Gebilde bezeichnet, die aus vier MHC-Molekülen und Antigen bestehen. An diese Gebilde binden T-Zellen tausend mal besser als an die einzelnen Komplexe. Die Tetramere binden dabei an die entsprechenden T-Zellrezeptoren. Dies entspricht der T-Zell vermittelten, sekundären Immunantwort durch Erkennung von zellgebundenen Antigenen, in Form von Peptiden, die von MHC-Komplexen auf Antigen-präsentierender Zellen gebunden sind.

Inzwischen können rekombinante, lösliche MHC-Moleküle hergestellt und an ein bekanntes Antigen gebunden werden, die durch Streptavidin tetramisiert werden können. Die so entstandenen peptid-spezifischen tetrameren MHC-Moleküle können mit Fluoreszenzfarbstoffen markiert werden und für Messungen im Durchflusszytometer eingesetzt werden. Anhand der Tetramer-Technik können Frequenzen antigen-spezifischer T-Zellen ausgemacht werden, um somit eine Aussage über die beteiligten Antigene in einem Krankheitsbild treffen zu können. Durch MHC-Moleküle ist das Sortieren und die Analyse von beispielsweise T-Zellen möglich, die Tumorantigene erkennen. Peptid-MHC-Tetramere besitzen daher großes therapeutisches Potential bei der Aufspürung antigen-spezifischer T-Zellen in humanen Autoimmun-Krankheiten am Beispiel der Artritis.

Eine Bindung der Tetramere an Partikel würde eine bessere Bindung von antigen-spezifischen T-Zellen an die Partikel gewährleisten, die dann wiederum aufgrund der Partikel von übrigen, nicht gebundenen Zellen, separiert werden können.

Reversible MHC-Peptid-Multimere, sog. Streptamere, sind eine neue Technologie zur Darstellung und Isolierung von cytotoxischen T-Lymphozyten. Im Gegensatz zu den bisher verwendeten Tetrameren, können sie von den T-Zellen wieder abgelöst werden und beeinflussen somit nicht die Funktion der Zellen.

Bindet man diese Partikel an magnetische Kügelchen, so haben aufgrund einer immunspezifischen Reaktion an diese Partikel gekoppelte biologische Partikel dann als gebundene biologische Partikel ebenfalls magnetische, bevorzugt superparamagnetische oder ferromagnetische Eigenschaften. Daher können durch Verwendung von Magneten, beispielsweise Elektromagneten oder Permanentmagneten, solchermaßen an mit magnetischen Partikeln gekoppelte Antikörper gebundene biologische Partikel separiert und isoliert werden.

Aus dem Stand der Technik (WO 01/24850 A1) ist darüber hinaus eine extrakorporale Vorrichtung zum Entfernen von Toxinen aus dem menschlichen Blut mit Hilfe von die Toxine absorbierenden magnetischen Partikeln bekannt. Hierbei wird das Blut dem Patienten entnommen und in einer Mixkammer mit einer Suspension der magnetischen Partikel vermischt. Anschließend erfolgt eine Abtrennung der Partikel in der Kammer mit Hilfe eines Magnetmechanismus. Das von den Toxinen/magnetischen Partikeln befreite Blut wird anschließend sicherheitshalber über eine magnetische Falle sowie über einen Sensorabschnitt wieder in den Körper geleitet. Hierbei sind nach dem Mechanismus eine Abzweigung und eine Filtervorrichtung zum Entfernen von Wasser vorgesehen. In der eigentlichen Separationsvorrichtung sind in Flussrichtung hintereinander zwei Magnete mit entgegengesetzter Feldlinienrichtung vorgesehen.

Der Stand der Technik (US 2003/0044832 A1) kennt darüber hinaus eine immunomagnetische Separationsvorrichtung, bei der die Abtrennung mittels eines seitlich neben der Separationsvorrichtung angeordneten Magneten geschieht. Hierbei werden die immunomagnetischen Partikel mittels des Magneten von einem laminaren Strom in einen benachbarten laminaren Strom gezogen und separat abgeführt.

Aufgabe der vorliegenden Erfindung ist es, eine im Durchflussverfahren arbeitende Separationsvorrichtung bzw. ein entsprechendes Separationsverfahren zur Verfügung zu stellen, mit der bzw. mit dem eine automatische und kontinuierliche Isolation biologischer Partikel auf einfache Art und Weise möglich ist.

Zur Lösung dieser Aufgabe verwendet die erfindungsgemäße Vorrichtung einen einfachen mikrofluidischen Kanal mit zwei Einlässen bzw. Einlasskanälen und zwei Auslässen bzw. zwei Auslasskanälen sowie ein oder mehrere Magnete, beispielsweise Elektromagnete oder Permanentmagnete. Unter einem Kanal (dies gilt sowohl für den Durchströmungskanal als auch für die in ihn einmündenden Einlasskanäle und die aus ihm wegführenden Abführkanäle) wird im Folgenden ein von einem Fluid durchströmbares Volumen samt der dieses Volumen umgebenden Wandung verstanden.

Durch den ersten Einlasskanal wird eine Flüssigkeit, welche verschiedene biologische und/oder auch nicht biologische Materialien (inklusive der über die spezifische Immunreaktion zu bestimmenden biologischen Partikel) enthält, in den mikrofluidischen Durchströmungskanal eingeleitet. Durch den anderen Einlasskanal wird eine Flüssigkeit, welche die immunomagnetischen Partikel, die zur spezifischen Bindung an das zu bestimmende biologische Material ausgebildet sind, eingeleitet. Die spezifische Bindung kann dadurch erreicht werden, dass das mittels der Immunreaktion zu separierende biologische Material ein Antigen ist und dass die immunomagnetischen Partikel ferromagnetische oder superparamagnetische Kügelchen sind, welche an den entsprechenden Antikörper oder an antigenspezifische Tetramere oder Streptamere gebunden sind (Antigen-Antikörper/Tetramer/Streptamer-Reaktion).

Die rheologischen Eigenschaften der beiden Flüssigkeiten sowie die geometrischen Verhältnisse (insbesondere die Querschnittsflächen der beiden Einlasskanäle sowie die Querschnittsfläche des Durchströmungskanals) sind nun so ausgebildet, dass die durch die beiden Einlasskanäle zugeführten Flüssigkeitsströme sich im Durchströmungskanal nicht vermischen (bis auf Diffusionsprozesse). Dies kann auch dadurch erreicht werden, dass zwischen dem Bereich der Einlasskanäle und dem Bereich der Auslasskanäle im Durchströmungskanal eine Trennwand derart vorgesehen ist, dass lediglich im Bereich der Einlasskanäle und im Bereich der Auslasskanäle die jeweils zugeführten bzw. abgeleiteten Flüssigkeitsströme sich kontaktieren. Dadurch werden unerwünschte Diffusionseffekte zwischen den Strömen minimiert und eine noch reinere Separation der abzutrennenden biologischen Partikel möglich.

Mit Hilfe des ersten Magneten (bzw. dessen magnetischen Feldes oder Feldgradienten) erhalten nun im Bereich der Einlasskanäle die immunomagnetischen Partikel aufgrund ihres ferromagnetischen oder superparamagnetischen Charakters eine Geschwindigkeitskomponenten senkrecht zur Flussrichtung. Die immunomagnetischen Partikel können dadurch die Grenze der beiden laminaren Strömungen überwinden bzw. werden vom einen Flüssigkeitsstrom in den anderen Flüssigkeitsstrom gezogen. In letzterem befinden sich dann die zu separierenden spezifischen biologischen Partikel, an die die immunomagnetischen Partikel binden. Durch den geeignet angeordneten ersten Magneten oder einen weiteren stromabwärts angeordneten, zweiten Magneten werden im Bereich der Auslasskanäle die zumindest teilweise an die zu separierenden biologischen Partikel gebundenen immunomagnetischen Partikel dann durch Anwendung eines entgegengesetzt gerichteten Magnetfeldes bzw. Feldgradienten wieder in den ursprünglichen Flüssigkeitsstrom zurückgezogen. Der die an das zu separierende biologische Material gebundenen immunomagnetischen Partikel enthaltende Flüssigkeitsstrom wird dann über einen der Auslasskanäle abgeführt, während der andere Flüssigkeitsstrom (welcher die restlichen biologischen und/oder nicht biologischen Materialien und ungebundene Partikel des zu separierenden biologischen Materials enthält) mit Hilfe des anderen Auslasskanals abgeführt wird.

Entscheidend ist, dass im mikrofluidischen Durchströmungskanal aufgrund der dort herrschenden Verhältnisse (rheologische Eigenschaften der Flüssigkeiten sowie insbesondere die Querschnittsfläche des Kanals) laminare Strömungsverhältnisse vorliegen. Aus diesem Grunde vermischen sich die beiden Flüssigkeitsströme nicht bzw. nicht wesentlich. Daher überwinden im wesentlichen nur die immunomagnetischen Partikel mit Hilfe des ersten magnetischen Feldes die Grenze zwischen den beiden Flüssigkeitsströmen und die gebundenen sowie die ungebunden verbliebenen immunomagnetischen Partikel überwinden mit Hilfe des magnetischen Feldes des zweiten Elektromagneten die Grenzen der beiden Flüssigkeitsströmungen erneut in umgekehrter Richtung. Die immunomagnetischen Partikel werden somit getrennt zu der die zu separierenden biologischen Partikel enthaltenden Flüssigkeit eingeleitet, wechseln dann für eine bestimmte Zeit aus ihrem Flüssigkeitsstrom in den benachbarten Flüssigkeitsstrom der biologischen Materialien, binden dort an die zu separierenden biologischen Partikel und werden anschließend mit Hilfe des zweiten magnetischen Feldes mit den an sie gebundenen biologischen Partikeln wieder zurück in ihren ursprünglichen Strom gezogen. Die die nicht gebundenen biologischen Partikel sowie andere biologische Materialien enthaltende Flüssigkeit wird dann über den einen Auslass bzw. Abführkanal abgeleitet, während die gebundenen und somit isolierten biologischen Partikel aus dem anderen Auslass abgeleitet werden können.

In einer vorteilhaften Ausgestaltungsvariante kann die erfindungsgemäße Vorrichtung mit einer Reaktionskammer versehen sein. Diese ist am Durchströmungskanal auf der Seite des die biologischen Materialien enthaltenden Flüssigkeitsstroms bzw. des ersten Magneten angeordnet und dient der Verlängerung der Zeit, die dieser Flüssigkeitsstrom für das Durchströmen des Durchströmungskanals benötigt. Die Reaktionskammer ist in Strömungsrichtung zwischen den beiden Magneten angeordnet, so dass sich eine erhöhte Aufenthaltsdauer der in den Strom gezogenen immunomagnetischen Partikel ergibt und somit eine höhere Wahrscheinlichkeit der Bindung der immunomagnetischen Partikel an das spezifische biologische Material.

Die vorstehend beschriebene immunomagnetische Separationsvorrichtung weist eine Reihe von Vorteilen auf:
- Die Vorrichtung ermöglicht eine einfache Isolation, ohne zusätzliche Mischungs-, Inkubations- und Waschschritte, welche ansonsten per Hand durchgeführt werden müssten und daher zeitaufwendig sind sowie zusätzliche Flüssigkeiten erfordern. Mit der Vorrichtung ist eine automatische und kontinuierliche Partikelisolation bzw. Separation möglich, wobei nur geringe oder gar keine Mengen an Puffer-, Transport- und/oder Verdünnungsflüssigkeit notwendig sind. Probenverdünnungslösungen und extra Pufferlösungen sind somit nicht notwendig in der vorliegenden Vorrichtung.
- Die gebundenen biologischen Partikel können somit ohne zusätzliche Auswaschprozesse aus der ursprünglichen, verschiedene biologische Materialien enthaltenden Mischflüssigkeit isoliert und separiert werden. Die separierten biologischen Partikel werden über einen getrennten Abführkanal erhalten.
- Die antikörpergekoppelten magnetischen Partikel bzw. immunomagnetischen Partikel können ihrem zugehörigen Einlasskanal direkt zugeführt werden, ohne dass zusätzlich ein Vormischungsschritt oder ein Inkubationsschritt notwendig ist.
- Die Vorrichtung kann mit einer automatischen Kontrollvorrichtung zur Steuerung der Magnetfeldstärken bzw. Magnetfeldgradienten versehen sein. Die Vorrichtung kann darüberhinaus auch mit einer Regelungsvorrichtung versehen sein, welche die Steuerung der Durchflussgeschwindigkeit im Durchströmungskanal bzw. der pro Zeiteinheit durchströmenden Flüssigkeitsmengen regelt. Die Regelung der Durchflussgeschwindigkeit bzw. die pro Zeiteinheit durchströmende Flüssigkeitsmenge kann auch durch geeignete Regelungsvorrichtungen im Bereich der Einlasskanäle und/oder Auslasskanäle erfolgen. Somit ist auf einfache und kontrollierte Art und Weise die Markierung bzw. Bindung der biologischen Partikel und ihre Isolierung möglich.

Die erfindungsgemäße Vorrichtung kann als medizinisches Diagnosesystem außerhalb des menschlichen oder tierischen Körpers eingesetzt werden. Auf eben solche Art und Weise kann die erfindungsgemäße Vorrichtung auch zu therapeutischen Zwecken, z.B. zum Isolieren bestimmter Zellarten aus Blut oder Patientengewebe und ähnlichem, eingesetzt werden. Die Vorrichtung kann somit insbesondere implantierbar sein und kontinuierliche Separations- bzw. Messprozesse ermöglichen. Insbesondere für eine implantierbare Vorrichtung können diese sowie ihre Kontrollelektronik integriert gefertigt werden und somit eine Größe aufweisen, welche zur Implantierung geeignet ist, und auf kosteneffiziente Art und Weise gefertigt werden. Wird die erfindungsgemäße Vorrichtung außerhalb des menschlichen oder tierischen Körpers eingesetzt, so kann diese als Laborgerät ausgestaltet sein. Das Laborgerät kann dann zur Zellseparation z.B. von Blutproben, gemischten Zellpopulationen (z.B. aus Patientengewebe) oder von Zellen mit bestimmten Charakteristiken (z.B. bestimmten Oberflächenmarkern oder physiologischen Zuständen) verwendet werden.

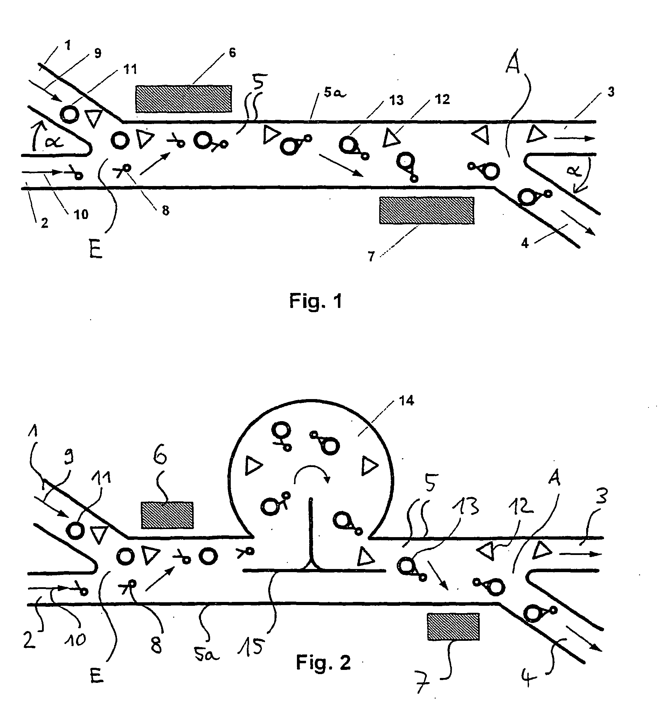

Die erfindungsgemäße Vorrichtung kann, wie in einem der beiden folgenden Beispiele dargestellt, aufgebaut sein oder verwendet werden.
Es zeigt Figur 1 eine erste erfindungsgemäße immunomagnetische Separationsvorrichtung;
Figur 2 eine zweite erfindungsgemäße immunomagnetische Separationsvorrichtung mit einer Reaktionskammer;
Figur 3 eine dritte erfindungsgemäße immunomagnetische Separationsvorrichtung; und
Figur 4 eine weitere vierte erfindungsgemäße immunomagnetische Separationsvorrichtung.

In den den Beispielen entsprechenden, im folgenden beschriebenen Figuren werden für gleiche oder identische Bestandteile der Vorrichtung identische Bezugszeichen verwendet.

Figur 1 zeigt eine immunomagnetische Separationsvorrichtung. Die Figur 1 zeigt einen Schnitt durch eine erfindungsgemäße immunomagnetische Separationsvorrichtung in einer zentralen, durch den Schwerpunkt der Vorrichtung verlaufenden Ebene. Die Vorrichtung weist einen mikrofluidischen Durchströmungskanal 5 mit einem Einmündungsbereich E und einem stromabwärts davon angeordneten Abführungsbereich A auf. Im Einmündungsbereich E münden ein erster Einlasskanal 1 und ein zweiter Einlasskanal 2 in den Durchströmungskanal 5. Der zweite Einlasskanal mündet hierbei in Richtung der Strömungsrichtung durch den Durchströmungskanal 5 ein. Der erste Einlasskanal 1 mündet unter einem Winkel von α = 30° zur Durchströmungsrichtung durch den Durchströmungskanal 5 ein. Im Abführungsbereich A führen zwei Abführkanäle 3 und 4 aus dem Durchströmungskanal 5 weg. Der Abführkanal 3 führt hierbei in Richtung der Strömungsrichtung durch den Durchströmungskanal 5 weg, der Abführkanal 4 führt unter einem Winkel von α = 30° zu dieser Richtung weg. Der Durchmesser der Einlasskanäle 1, 2 und der Abführkanäle 3, 4 senkrecht zur jeweiligen Durchströmungsrichtung beträgt in etwa die Hälfte des Durchmessers des Durchströmungskanals 5 senkrecht zu dessen Durchströmungsrichtung.

Stromabwärts vom Einmündungsbereich E ist außerhalb des Durchströmungskanals 5 und seitlich neben dem Durchströmungskanal 5 ein erster Elektromagnet 6 angeordnet. Stromabwärts dieses ersten Elektromagneten 6 und unmittelbar stromaufwärts des Abführbereiches A ist ein zweiter Elektromagnet 7 ebenfalls außerhalb des Durchströmungskanals 5 und seitlich neben dem Durchströmungskanal 5 angeordnet. Die beiden Elektromagnete 6 und 7 sind auf unterschiedlichen Seiten, im vorliegenden Fall auf gegenüberliegenden Seiten des Durchströmungskanals 5 angeordnet.

Die beiden Elektromagneten 6 und 7 können alternativ hierzu jedoch auch zumindest teilweise in die Wandung 5a des Durchströmungskanals 5 integriert werden. In diesem Fall werden die beiden Elektromagneten 6 und 7 dann auf im Wesentlichen gegenüberliegenden Seiten in die Wandung 5a des Durchströmungskanals 5 integriert. Es ist jedoch auch möglich, die beiden Elektromagneten 6 und 7 vollständig innerhalb des Durchströmungskanals 5 bzw. innerhalb der Wandung 5a des Durchströmungskanals 5 im von der Wandung 5a umschlossenen Volumen des Durchströmungskanals 5 anzuordnen. Die beiden Elektromagneten 6 und 7 werden dann innerhalb des Durchströmungskanals 5 ebenfalls im wesentlichen auf gegenüberliegenden Seiten des Durchströmungskanals angeordnet (dies geschieht bevorzugt im Wandbereich des Durchströmungskanals oder auch so, dass die Elektromagneten 6 und 7 auf der Kanalinnenwand aufgesetzt bzw. dort befestigt werden). Es ist jedoch auch möglich, jeweils eine unterschiedliche der beschriebenen Varianten für den Elektromagneten 6 und den Elektromagneten 7 einzusetzen: So kann der Elektromagnet 6 vollständig außerhalb der Wandung 5a des Kanals angeordnet sein, während der Elektromagnet 7 auf der gegenüberliegenden Seite des Durchströmungskanals 5 in dessen Wandung integriert ist oder innerhalb des Kanals auf der gegenüberliegenden Seite auf die Innenoberfläche der Wandung 5a aufgesetzt ist.

Die Einlasskanäle 1, 2, die Abführkanäle 3, 4, der Durchströmungskanal 5 sowie die beiden Elektromagneten 6 und 7 (bzw. die entsprechenden Zentralachsen bzw. Schwerpunkte) sind im vorliegenden Fall in einer Ebene angeordnet.

Entscheidend ist nun, dass die Verhältnisse in den Strömungskanälen aufgrund ausreichend kleiner Durchmesser der Einlasskanäle, Auslasskanäle und des Durchströmungskanals sowie aufgrund von ausreichend kleinen Strömungsgeschwindigkeiten so ausgestaltet sind, dass sich zwei getrennt übereinander gleitende Flüssigkeitsströme bzw. Flüssigkeitsschichten ausbilden können, ohne miteinander zu verwirbeln (laminare Strömung). Wird somit durch den ersten Einlasskanal 1 eine verschiedene biologische Partikel 11, 12 enthaltende Mischflüssigkeit 9 eingeleitet und durch den zweiten Einlasskanal 2 eine Flüssigkeit 10, welche immunomagnetische Partikel 8 enthält, so durchmischen sich die beiden eingeleiteten Flüssigkeitsströmungen (bis auf Diffusionsprozesse) nicht, sondern gleiten in Richtung des Abführungsbereiches A als getrennte Flüssigkeitsschichten parallel zueinander. Der erste Flüssigkeitsstrom der Mischflüssigkeit 9 wird dann ohne sich mit dem zweiten Flüssigkeitsstrom 10 der immunomagnetischen Partikel 8 zu vermischen, über den ersten Abführkanal 3 abgeführt. Der zweite Flüssigkeitsstrom 10 entsprechend über den zweiten Abführkanal 4.

Entscheidend ist also, dass im mikrofluidischen Durchströmungskanal 5 die durchströmenden Flüssigkeiten eine so geringe Reynolds-Zahl aufweisen, dass die Strömungsverhältnisse im Durchströmungskanal 5 als laminar angesehen werden können. Damit sind Trägheitseffekte, welche Turbulenzen und sekundäre Ströme bzw. Wirbel verursachen, vernachlässigbar und eine Vermischung ist lediglich aufgrund von Diffusionsprozessen möglich. Um dies zu gewährleisten, besitzt in dem dargestellten Fall der Mikro-Durchströmungskanal 5 eine Breite von 0,1 bis 0,3 mm und eine Höhe von 0,1 bis 0,2 mm (rechteckförmiger Durchströmungskanal, Breite und Höhe senkrecht zur Längsrichtung bzw. zur Durchströmungsrichtung). Die Gesamtdurchflussrate (geregelt über eine nicht dargestellte Regelungsvorrichtung) beträgt für den Mikro-Durchströmungskanal 5 zwischen 1 und 200 µl/min. Diese mikrofluidischen Strömungscharakteristika erfüllen die notwendigen Voraussetzungen für laminare Strömungsverhältnisse im Mikro-Durchströmungskanal 5. Aus diesem Grund vermischen sich die über den ersten Einlasskanal 1 eingeführte Mischflüssigkeit 9 und die über den zweiten Einlasskanal 2 eingeführte, die immunomagnetischen Partikel 8 enthaltende Flüssigkeit 10 im Durchströmungskanal 5 nicht, sondern bilden zwei getrennte Strömungsschichten aus. Somit werden auch die unterschiedlichen Partikel (biologische Partikel 11, 12 und immunomagnetische Partikel 8) eines jeden Flüssigkeitsstroms bei ausgeschalteten Elektromagneten 6, 7 nicht vermischt, sondern fließen kontinuierlich in ihrem jeweiligen Flüssigkeitsstrom bis zu ihrem jeweiligen Abführkanal 3 oder 4.

Neben den zu separierenden biologischen Partikeln 11 enthält im vorliegenden Fall die Mischflüssigkeit 9 weitere biologische (oder auch andere) Partikel 12, von denen die zu separierenden Partikel 11 getrennt werden sollen. Solche weiteren Partikel 12 müssen jedoch nicht vorhanden sein, so dass die vorliegende Erfindung auch zur Änderung der Konzentration der zu separierenden Partikel 11 im Flüssigkeitsstrom 9 eingesetzt werden kann. Wird nun der erste Elektromagnet 6 aktiviert, so werden die immunomagnetischen Partikel 8 einem elektromagnetischen Feld bzw. Feldgradienten unterworfen, welcher eine Kraft senkrecht zur Durchströmungsrichtung durch den Durchströmungskanal 5 und in Richtung auf den ersten Elektromagneten 6 hin ausübt. Hierdurch werden die immunomagnetischen Partikel 8 aus ihrem zweiten Flüssigkeitsstrom 10 über die Flüssigkeitsstromgrenze hinweg in den ersten Flüssigkeitsstrom 9 der Mischflüssigkeit gezogen. Die immunomagnetischen Partikel 8 vermischen sich damit mit den im Mischflüssigkeitsstrom 9 befindlichen Partikeln 11, 12, und können somit durch die spezifische Antigen-Antikörper-Reaktion an die zu separierenden Partikel 11 anbinden (so entstehen kombinierte bzw. gebundene Partikel 13, welche jeweils mindestens einen immunomagnetischen Partikel 8 und einen biologischen Partikel 11 aufweisen). Die Feldstärke bzw. die Gradientenstärke des Elektromagneten 6 kann so kontrolliert bzw. eingestellt werden, dass die erzeugten Kräfte gerade ausreichen, um die immunomagnetischen Partikel 8 vom zweiten Flüssigkeitsstrom 10 in den ersten Flüssigkeitsstrom 9 zu ziehen. Das magnetische Feld des Elektromagneten 6 (dies gilt ebenso für den Elektromagneten 7) kann hierbei pulsförmig oder sinusförmig moduliert werden. Die immunomagnetischen Partikel fließen dann frei mit einem ausgeglichenen Verhältnis zwischen der Strömungsgeschwindigkeit in Durchströmungsrichtung und der senkrecht dazu durch das magnetische Feld induzierten Geschwindigkeit.

Nachdem die immunomagnetischen Partikel 8 in den ersten Flüssigkeitsstrom der Mischflüssigkeit 9 gezogen worden sind, kombinieren sie, wie bereits beschrieben, durch eine immunspezifische Reaktion mit den zu separierenden biologischen Partikeln 11 zu den gebundenen Partikeln 13. Die Schmalheit bzw. die geringe Querschnittsfläche des Mikro-Durchströmungskanals 5 (ausreichend geringer Durchmesser) und ausreichend geringe Durchströmungsgeschwindigkeiten durch den Durchströmungskanal 5 erhöhen die Wahrscheinlichkeit, dass die einzelnen immunomagnetischen Partikel 8 an die zugehörigen biologischen Partikel 11 binden (Erhöhung der Zeit, die für die Immunreaktion zur Verfügung steht).

Stromabwärtsseitig zum ersten Elektromagneten 6 ist auf der diesem Magneten gegenüberliegenden Seite des Durchströmungskanals 5 nun unmittelbar vor dem Abführungsbereich A der zweite Elektromagnet 7 angeordnet. Mit Hilfe dieses zweiten Elektromagneten 7 werden nun die gebundenen Partikel 13 und auch immunomagnetische Partikel 8, die auf dem Strömungsweg zwischen dem Elektromagneten 6 und dem Elektromagneten 7 nicht an biologische Partikel 11 gebunden worden sind, vom ersten Flüssigkeitsstrom 9 wieder über die Flüssigkeitsstromgrenze in den zweiten Flüssigkeitsstrom 10 zurückgezogen. Dies geschieht über ein elektromagnetisches Feld bzw. einen Feldgradienten des Elektromagneten 7, welches bzw. welcher entgegengesetzt zum Feld bzw. Gradienten des ersten Elektromagneten 6 gerichtet ist. Die immunomagnetisch gebundenen bzw. gekennzeichneten biologischen Partikel 13 sowie die ungebundenen immunomagnetischen Partikel 8 bzw. der zweite Flüssigkeitsstrom 10 wird dann über den zweiten Abführkanal 4 abgeleitet. Der erste Flüssigkeitsstrom 9 bzw. die restlichen nicht gebundenen biologischen Partikel 11 sowie die anderen biologischen Materialien 12 werden über den ersten Abführkanal 3 abgeführt. Die (gebundenen) biologischen Partikel 11 bzw. 13 sind somit von den anderen biologischen Materialien 12 getrennt worden.

Figur 2 zeigt eine immunomagnetische Separationsvorrichtung, deren Grundaufbau der in Figur 1 gezeigten Separationsvorrichtung entspricht. In Strömungsrichtung nach dem ersten Elektromagneten 6 und vor dem zweiten Elektromagneten 7 weist der Durchströmungskanal 5 jedoch eine auf der Seite des ersten Elektromagneten 6 angeordnete Ausbuchtung (Reaktionskammer) 14 auf. Im vorliegenden Fall ist der Durchströmungskanal 5 mit der Reaktionskammer 14 einstückig ausgebildet. Die Reaktionskammer 14 kann jedoch auch als separates Bauteil an einer entsprechenden Öffnung im Durchströmungskanal 5 realisiert sein. In der gezeigten Schnittebene (Anordnungsebene der Einlasskanäle 1, 2, der Auslasskanäle 3, 4 sowie der beiden Elektromagneten 6, 7) weist die Reaktionskammer 14 einen Ω-förmigen Querschnitt auf. Auf Höhe der Reaktionskammer 14 ist ein im dargestellten Schnitt T-förmiger Strömungsbrecher 15 im Durchströmungskanal 5 angeordnet. Der Strömungsbrecher 15 ist in Strömungsrichtung auf Höhe der Kammer 14 so angeordnet, dass er lediglich in den ersten Flüssigkeitsstrom der Mischflüssigkeit 9 eingreift und diesen Flüssigkeitsstrom in die Reaktionskammer 14 ablenkt. Durch die aus dem Strömungsbrecher 15 und der Reaktionskammer 14 bestehende Reaktionsvorrichtung wird der Weg der ersten Flüssigkeitsströmung 9 durch den Durchströmungskanal 5 verlängert. Durch diese Reaktionsvorrichtung wird die Aufenthaltsdauer des ersten Flüssigkeitsstroms 9 im Durchströmungskanal 5 proportional zum Volumen der Reaktionskammer 14 erhöht. Hierdurch ist eine erhöhte Kontakteffizienz bzw. eine Verlängerung der Zeit, die den immunomagnetischen Partikeln 8 zur Bindung an die spezifischen biologischen Partikel 11 zur Verfügung steht, gegeben. Die Wahrscheinlichkeit, dass eine Immunreaktion stattfindet bzw. dass die immunomagnetischen Partikel 8 binden, wird somit erhöht. Die Separierungseffizienz wird somit durch die erhöhte Immunreaktionseffizient der Vorrichtung erhöht. Die vorgestellte Reaktionskammer 14 bedingt hohe Flussgeschwindigkeitsgradienten und eine gute Mikro-Durchmischung des ersten Flüssigkeitsstroms 9. Auch hierdurch wird die Bindungswahrscheinlichkeit der immunomagnetischen Partikel 8 erhöht. Entscheidend ist hierbei, dass die Reaktionsvorrichtung 14, 15 in Strömungsrichtung zwischen den beiden Elektromagneten 6 und 7 zur Verfügung gestellt wird, so dass der erste Flüssigkeitsstrom, wenn er bereits die eingezogenen immunomagnetischen Partikel 8 aufweist, in diese den Bindungszeitraum verlängernde Reaktionskammer 14 eingeleitet wird.

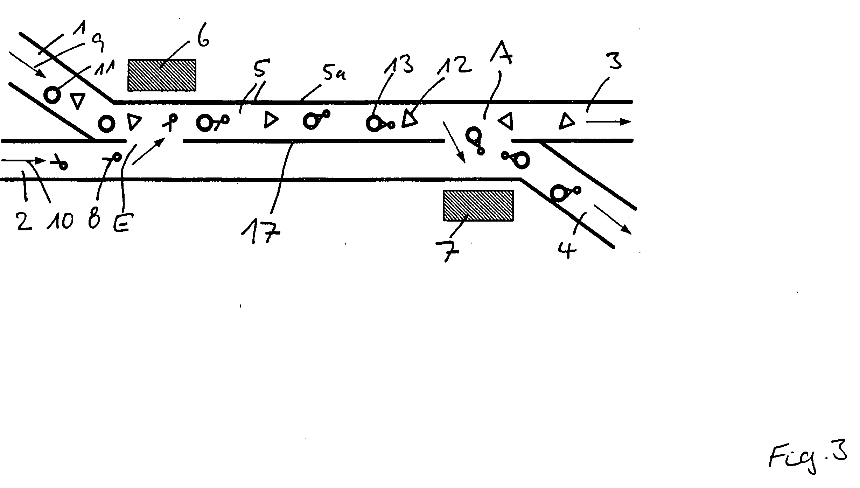

Figur 3 zeigt eine weitere erfindungsgemäße Separationsvorrichtung, die weitestgehend wie diejenige in Figur 1 ausgestaltet ist. Im Unterschied zu Figur 1 befindet sich nun jedoch zwischen dem Einmündungsbereich E und dem stromabwärts davon angeordneten Abführungsbereich A eine Trennwand 17, die die beiden Flüssigkeitsströme, die durch den Einlasskanal 1 bzw. den Einlasskanal 2 der Trennvorrichtung zugeleitet werden, voneinander separiert. So ist es lediglich im Bereich E der beiden Einlässe 1 und 2 möglich, dass die magnetischen Partikel aufgrund der durch den Magneten 6 ausgeübten magnetischen Kraft von dem einen Flüssigkeitsstrom in den anderen wechseln und derselbe Wechsel in umgekehrter Richtung im Bereich A erfolgt. Zwischen diesen beiden Bereichen E und A kann keine weitere Vermischung der Flüssigkeitsströme erfolgen, sodass in diesem Bereich lediglich eine Anlagerung zwischen immunomagnetischem Partikel und antigenbehaftetem Partikel erfolgt.

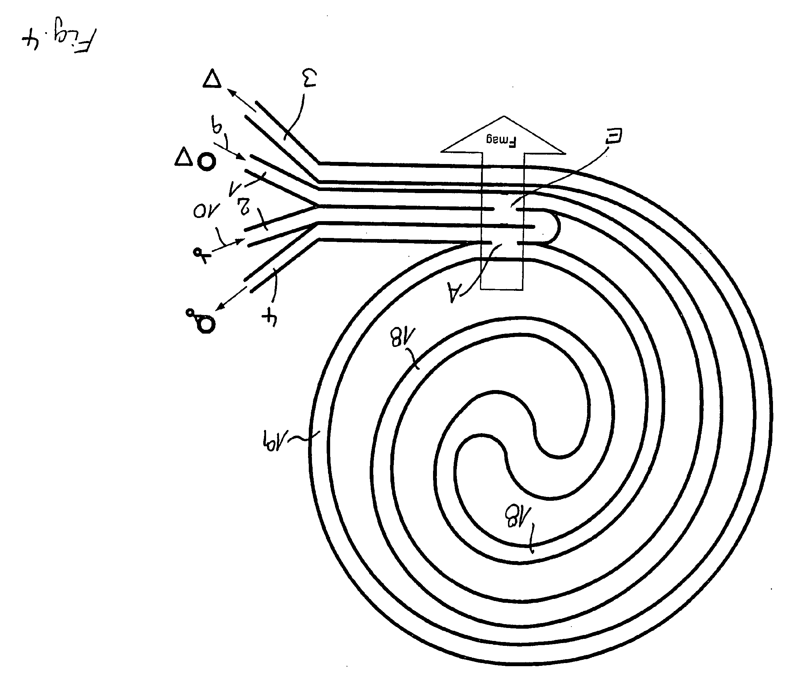

Figur 4 zeigt eine weitere erfindungsgemäße Separationsvorrichtung. Hier erfolgt die Zufuhr von immunomagnetischen Partikeln 11 über einen Einlasskanal 2 und die Zufuhr der Probe über einen Einlasskanal 1, die in einem mit E bezeichneten Bereich miteinander kommunizieren, sodass dort aufgrund eines angelegten Magnetfeldes Fmag die immunomagnetischen Partikel 11 in die Probe übertreten können. Das erzeugte Magnetfeld Fmag wird durch einen Pfeil dargestellt. Die Probe mit den immunomagnetischen Partikeln 11 wird dann in einer Spirale 18 über eine lange Strecke geführt, sodass dort die immunomagnetischen Partikel 11 an Antigene 8 ankoppeln können. Die Spirale 18 wird dann wieder zurückgeführt und trifft in einem Bereich A auf die zwischenzeitlich umgelenkte Flüssigkeit, die ursprünglich die immunomagnetischen Partikel 11 enthielt. In diesem Bereich A werden die Immunopartikel 8 beladenen Partikel 11 wiederum in den ursprünglichen Flüssigkeitsstrom durch das Magnetfeld Fmag zurückgezogen und anschließend über den Auslass 4 abgeführt. Die von den immunomagnetischen Partikeln 11 damit wieder weitgehend befreite Probe wird in einem großen Bogen 19 um die Spirale 18 herum geführt und zuletzt über den Auslass 3 abgeführt. Diese Anordnung hat den Vorteil, dass der Mischbereich zwischen den magnetischen Partikeln 11 und den Antikörpern 8 eine sehr lange Strecke aufweist. Weiterhin hat sie den Vorteil, dass lediglich ein Magnet benötigt wird, um das Magnetfeld im Bereich E und das Magnetfeld im Bereich A zu erzeugen und damit sämtliche Misch- und Trennvorgänge zu bewerkstelligen.

## Patentansprüche

1. Separationsvorrichtung
mit einem Durchströmungskanal (5) mit einer Wandung (5a), einem Einmündungsbereich (E) und einem stromabwärts davon angeordneten Abführungsbereich (A) und
mindestens einem Magneten (6, 7) zur Erzeugung eines magnetischen Feldes über zumindest einen Teil des Querschnitts des Durchströmungskanals (5),
wobei im Einmündungsbereich (E) zwei Einlasskanäle (1, 2) zur Zufuhr von Fluiden in den Durchströmungskanal (5) münden und wobei zwei Abführkanäle (3, 4) zum Abtransport von Fluiden aus dem Abführungsbereich (A) wegführen,
wobei ein erster Magnet (6) stromabwärts des Einmündungsbereiches (E) ein erstes Magnetfeld erzeugt,
wobei der erste Magnet (6) oder ein weiterer Magnet (7) stromabwärts des ersten Magnetfeldes und stromaufwärts des Abführungsbereiches (A) ein zweites Magnetfeld erzeugt, wobei das erste und das zweite Magnetfeld im Wesentlichen bezüglich der Strömungrichtung oder bezüglich ihrer Polarität entgegengesetzt sind, und
wobei der Durchströmungskanal und/oder die Einlasskanäle und/oder die Auslasskanäle so angeordnet und/oder so ausgebildet sind, dass im Durchströmungskanal bei Durchströmung von zur immunomagnetischen Separation einsetzbarer Fluide eine laminare Strömung oder eine Strömung mit einer Reynoldszahl R kleiner als der kritischen Reynoldszahl Rₖᵣᵢₜ erzeugbar ist und dass im Durchströmungskanal auf einer ersten Seite ein erster Flüssigkeitsstrom ausbildbar ist und dass im Durchströmungskanal auf einer zweiten zur ersten Seite unterschiedlichen Seite ein zweiter, vom ersten Flüssigkeitsstrom bis auf Diffusionsprozesse getrennter Flüssigkeitsstrom ausbildbar ist.

2. Separationsvorrichtung nach dem vorhergehenden Anspruch,
***dadurch gekennzeichnet, dass***
der Durchströmungskanal und/oder die Einlasskanäle und/oder die Auslasskanäle in Bezug auf ihren Querschnitt senkrecht zur Durchströmungsrichtung räumlich aus so ausgebildet sind, dass im Durchströmungskanal bei Durchströmung von zur immunomagnetischen Separation einsetzbarer Fluide eine laminare Strömung oder eine Strömung mit einer Reynoldszahl R kleiner als der kritischen Reynoldszahl Rₖᵣᵢₜ erzeugbar ist und dass im Durchströmungskanal auf der Seite des ersten Magnets (6) ein erster Flüssigkeitsstrom ausbildbar ist und dass im Durchströmungskanal auf der Seite des zweiten Magnets (7) ein zweiter, vom ersten Flüssigkeitsstrom bis auf Diffusionsprozesse getrennter Flüssigkeitsstrom ausbildbar ist.

3. Separationsvorrichtung nach einem der vorhergehenden Ansprüche,
***gekennzeichnet durch***
einen ersten Magneten (6) und einen zweiten Magneten (7) zur Erzeugung von magnetischen Feldern über zumindest einen Teil des Querschnitts des Durchströmungskanals (5),
wobei im Einmündungsbereich (E) zwei Einlasskanäle (1, 2) zur Zufuhr von Fluiden in den Durchströmungskanal (5) münden und wobei zwei Abführkanäle (3, 4) zum Abtransport von Fluiden aus dem Abführungsbereich (A) wegführen,
wobei der erste Magnet (6) stromabwärts des Einmündungsbereiches (E) seitlich außerhalb des Durchströmungskanals (5) oder zumindest teilweise integriert in die Wandung (5a) des Durchströmungskanals (5) oder innerhalb der Wandung (5a) im Durchströmungskanal angeordnet ist,
wobei stromabwärts des ersten Magneten (6) und stromaufwärts des Abführungsbereiches (A) der zweite Magnet (7) seitlich außerhalb des Durchströmungskanals (5) oder zumindest teilweise integriert in die Wandung (5a) des Durchströmungskanals (5) oder innerhalb der Wandung (5a) im Durchströmungskanal angeordnet ist, und
wobei der erste und der zweite Magnet auf im wesentlichen gegenüberliegenden Seiten des Durchströmungskanals (5) außerhalb oder innerhalb des Durchströmungskanals angeordnet bzw. in dessen Wandung (5a) integriert sind.

4. Separationsvorrichtung nach einem der vorhergehenden Ansprüche,
***dadurch gekennzeichnet, dass***
die zwei Einlasskanäle (1, 2) und die zwei Abführkanäle (3, 4) sowie bevorzugt auch der erste und der zweite Magnet im Wesentlichen in einer Ebene in Strömungsrichtung des Durchströmungskanals (5) angeordnet sind.

5. Separationsvorrichtung nach einem der vorhergehenden Ansprüche,
***dadurch gekennzeichnet, dass***
mindestens einer der Einlasskanäle (1, 2) und/oder mindestens einer der Auslasskanäle (3, 4) im wesentlichen in Strömungsrichtung des Durchströmungskanals (5) oder unter einem Winkel α mit 0 < α < 180°, insbesondere mit 0 < α < 90°, insbesondere mit 0 < α < 45°, geneigt hierzu einmündet bzw. wegführt.

6. Separationsvorrichtung nach einem der vorhergehenden Ansprüche,
***dadurch gekennzeichnet, dass***
jeweils einer der Einlasskanäle und einer der Auslasskanäle senkrecht zur Strömungsrichtung des Durchströmungskanals gesehen auf derselben Seite des Durchströmungskanals angeordnet sind.

7. Separationsvorrichtung nach einem der vorhergehenden Ansprüche,
***dadurch gekennzeichnet, dass***
der erste und/oder der zweite Magnet (6, 7) ein Permanentmagnet oder ein Elektromagnet ist.

8. Separationsvorrichtung nach dem vorhergehenden Anspruch,
***dadurch gekennzeichnet, dass***
die Feldstärke und/oder der Feldgradient des Elektromagneten zeitlich und/oder örtlich variierbar ist oder konstant haltbar ist.

9. Separationsvorrichtung nach einem der vorhergehenden Ansprüche,
***dadurch gekennzeichnet, dass***
der Durchströmungskanal ein Mikrofluidkanal, insbesondere mit einer Querschnittsfläche senkrecht zur Durchströmungsrichtung von über 0.002 mm² und/oder unter 1 mm², bevorzugt von über 0.01 mm² und/oder unter 0.06 mm² ist.

10. Separationsvorrichtung nach einem der vorhergehenden Ansprüche,
***dadurch gekennzeichnet, dass***
der Durchströmungskanal ein im Querschnitt im wesentlichen kreisförmiges, elliptisches, rechteckförmiges oder quadratisches Rohr ist.

11. Separationsvorrichtung nach einem der vorhergehenden Ansprüche,
***gekennzeichnet durch***
eine in Strömungsrichtung nach dem ersten Magnet und vor dem zweiten Magnet angeordnete, strömungswegverlängernde Reaktionsvorrichtung (14, 15).

12. Separationsvorrichtung nach dem vorhergehenden Anspruch,
***dadurch gekennzeichnet, dass***
die strömungswegverlängernde Reaktionsvorrichtung eine bevorzugt im wesentlichen auf der Seite des ersten Magnets (6) angeordnete Reaktionskammer (14) und einen im Innern des Durchströmungskanals (5) angeordneten Strömungsbrecher (15) aufweist.

13. Separationsvorrichtung nach dem vorhergehenden Anspruch,
***dadurch gekennzeichnet, dass***
mit dem Strömungsbrecher (15) der erste Flüssigkeitsstrom in die Reaktionskammer (14) lenkbar ist.

14. Immunomagnetische Separationsvorrichtung nach dem vorhergehenden Anspruch,
***dadurch gekennzeichnet, dass***
die Reaktionskammer (14) in einer Ebene parallel zur Strömungsrichtung des Durchströmungskanals einen im wesentlichen Ω-förmigen, halbkreisförmigen oder trapezförmigen Querschnitt aufweist und/oder dass der Strömungsbrecher (15) in dieser Ebene im wesentlichen einen dreiecksförmigen oder T-förmigen Querschnitt aufweist.

15. Separationsvorrichtung nach einem der drei vorhergehenden Ansprüche,
***dadurch gekennzeichnet*, *dass***
die Reaktionskammer (14) als Auswölbung der Wandung (5a) des Durchströmungskanals (5) bzw. einstückig mit diesem ausgebildet ist oder dass die Reaktionskammer (14) als separates, an einer Öffnung der Wandung (5a) des Durchströmungskanals (5) angeordnetes Bauteil ausgebildet ist.

16. Separationsvorrichtung nach einem der vorhergehenden Ansprüche,
***gekennzeichnet durch***
eine Kontrollvorrichtung, insbesondere eine Kontrollelektronik zur Steuerung des ersten und/oder zweiten Magneten (6, 7) und/oder eine Regelungsvorrichtung zur Regelung der Durchflussgeschwindigkeit im Durchströmungskanal (5) und/oder in den Einlasskanälen (1, 2) und/oder Abführkanälen (3, 4).

17. Separationsvorrichtung nach einem der vorhergehenden Ansprüche,
***dadurch gekennzeichnet, dass***
die Separationsvorrichtung in den menschlichen oder tierischen Körper implantierbar ist oder dass die Separationsvorrichtung außerhalb des menschlichen oder tierischen Körpers, insbesondere als Laborgerät, einsetzbar ist.

18. Separationsvorrichtung nach einem der vorhergehenden Ansprüche,
***dadurch gekennzeichnet, dass***
in dem Durchströmungskanal (5) in Strömungsrichtung des Fluids zwischen dem Bereich der Einlasskanäle (1, 2) und dem Bereich der Abführkanäle (3, 4) zumindest bereichsweise eine Trennwand angeordnet ist, die eine Vermischung benachbarter, getrennt voneinander eingeleiteter Fluidströme verhindert.

19. Separationsanordnung mit
einer immunomagnetischen Separationsvorrichtung und
einem eine Mehrzahl von immunomagnetischen, insbesondere antikörpergekoppelten oder mit antigenspezifischen Tetrameren gekoppelten Partikeln (8) aufweisenden Fluid, insbesondere einer Flüssigkeit,
***dadurch gekennzeichnet, dass***
die immunomagnetische Separationsvorrichtung nach einem der vorhergehenden Ansprüche ausgebildet ist.

20. Separationsanordnung nach dem vorhergehenden Anspruch,
***dadurch gekennzeichnet,* dass**
die Partikel (8) ferromagnetische und/oder superparamagnetische Eigenschaften und/oder im wesentlichen Kugelform aufweisen.

21. Separationsanordnung nach einem der beiden vorhergehenden Ansprüche,
***dadurch gekennzeichnet, dass***
der Durchströmungskanal (5) und/oder die Einlasskanäle (1, 2) und/oder die Auslasskanäle (3, 4) der Separationsvorrichtung so ausgebildet sind und/oder dass das Fluid bzw. die Flüssigkeit eine Viskosität, Dichte, Temperatur und mittlere Strömungsgeschwindigkeit im Durchströmungskanal (5) so aufweist, dass im Durchströmungskanal eine laminare Strömung bzw. eine Strömung mit einer Reynoldszahl R kleiner als der kritischen Reynoldszahl Rₖᵣᵢₜ vorliegt.

22. Separationsanordnung nach einem der drei vorhergehenden Ansprüche,
***dadurch gekennzeichnet, dass***
die Durchströmungsrate des die Partikel (8) aufweisenden Fluids bzw. der die Partikel aufweisenden Flüssigkeit durch den Durchströmungskanal über 0.1 µl/min und/oder unter 2000 µl/min, insbesondere über 1 µl/min und/oder unter 200 µl/min, beträgt.

23. Separationsanordnung nach einem der vier vorhergehenden Ansprüche,
***dadurch gekennzeichnet, dass***
die mittlere Durchströmungsgeschwindigkeit des die Partikel (8) aufweisenden Fluids bzw. der die Partikel aufweisenden Flüssigkeit im Durchströmungskanal über 0.03 mm/s und/oder unter 3000 mm/s, insbesondere über 0.3 mm/s und/oder unter 300 mm/s, beträgt.

24. Isolationsverfahren zur Isolation eines bestimmten biologischen Materials (11) außerhalb des menschlichen oder tierischen Körpers insbesondere eines Antigens, aus einem neben diesem biologischen Material (11) gegebenenfalls weitere biologische und/oder andere Materialien (12) aufweisenden ersten Fluid (9), insbesondere einer Flüssigkeit, mit Hilfe eines eine Mehrzahl von immunomagnetischen, insbesondere mit den zu diesem Antigen spezifischen Antikörpern, Tetrameren und/oder Streptameren gekoppelten Partikeln (8) aufweisenden zweiten Fluids (10), insbesondere einer Flüssigkeit,
wobei das erste Fluid (9) und gleichzeitig das zweite Fluid (10) so in einen Durchströmungskanal (5) eingeleitet werden, dass sich im Durchströmungskanal (5) zwischen den beiden eingeleiteten Fluidströmen laminare Strömungsverhältnisse ausbilden und das erste Fluid (9) in einem ersten Flüssigkeitsstrom und das zweite Fluid (10) in einem zweiten, benachbarten Flüssigkeitsstrom durch den Durchströmungskanal fließt,
wobei mit Hilfe eines ersten magnetischen Feldes die immunomagnetischen Partikel (8) zumindest teilweise aus dem zweiten Flüssigkeitsstrom in den ersten Flüssigkeitsstrom gezogen werden, dort anschließend zur Bindung an das bestimmte biologische Material (11) über einen Bindungszeitraum entsprechend einem Teil des zum Durchfließen des Durchströmungskanals benötigten Zeitraums belassen werden, bevor die gegebenenfalls zumindest teilweise an das bestimmte biologische Material gebundenen immunomagnetischen Partikel (8, 13) mit Hilfe eines zweiten magnetischen Feldes zumindest teilweise vom ersten Flüssigkeitsstrom in den zweiten Flüssigkeitsstrom gezogen werden,
und wobei die beiden Flüssigkeitsströme getrennt aus dem Durchströmungskanal (5) abgeführt werden.

25. Isolationsverfahren nach dem vorhergehenden Anspruch,
***dadurch gekennzeichnet, dass***
eine immunomagnetische Separationsvorrichtung oder eine immunomagnetische Separationsanordnung nach einem der Ansprüche 1 bis 23 verwendet wird.

26. Isolationsverfahren nach einem der beiden vorhergehenden Ansprüche,
***dadurch gekennzeichnet, dass***
die Feldstärke und/oder die Gradientenstärke des ersten und/oder zweiten magnetischen Feldes so gewählt wird, dass sie für den Übergang der immunomagnetischen Partikel (8) aus dem zweiten in den ersten Flüssigkeitsstrom bzw. für den Übergang der zumindest teilweise an das bestimmte biologische Material gebundenen immunomagnetischen Partikel (13) aus dem ersten in den zweiten Flüssigkeitsstrom gerade ausreichend ist.

27. Isolationsverfahren nach einem der drei vorhergehenden Ansprüche,
***dadurch gekennzeichnet, dass***
das erste und/oder zweite magnetische Feld gepulst erzeugt wird oder sinusförmig moduliert wird.

28. Immunomagnetisches Isolationsverfahren nach einem der vier vorhergehenden Ansprüche,
***dadurch gekennzeichnet, dass***
der Bindungszeitraum **dadurch** erhöht wird, dass der Strömungsweg des ersten Flüssigkeitsstroms durch den Durchströmungskanal verlängert wird.

29. Verwendung einer immunomagnetischen Separationsvorrichtung und/oder einer immunomagnetischen Separationsanordnung und/oder eines immunomagnetischen Isolationsverfahrens nach einem der vorhergehenden Ansprüche zur medizinischen Diagnose oder Therapie außerhalb des menschlichen oder tierischen Körpers.

## Claims

1. A separation device
comprising a flow channel (5) having a wall (5a), an intake region (E) and a discharge region (A) arranged downstream thereof and at least one magnet (6, 7) for generating a magnetic field over at least part of the cross-section of the flow channel (5),
wherein two inlet channels (1, 2) lead into the intake region (E) for the supply of fluids into the flow channel (5) and wherein two outlet channels (3, 4) lead out of the discharge region (A) for the removal of fluids,
wherein a first magnet (6) generates a first magnetic field downstream of the intake region (E),
wherein the first magnet (6) or a further magnet (7) generates a second magnetic field downstream of the first magnetic field and upstream of the discharge region (A), wherein the first and the second magnetic field are substantially opposed with respect to the direction of flow or with respect to their polarity, and
wherein the flow channel and/or the inlet channels and/or the outlet channels are arranged and/or formed so that, when fluids which are usable for immunomagnetic separation flow through the flow channel, a laminar flow or a flow with a Reynolds number R smaller than the critical Reynolds number R_{crit} can be generated therein and so that a first fluid flow can be formed in the flow channel on a first side and a second fluid flow which is separate from the first fluid flow except for diffusion processes can be formed in the flow channel on a second side which is different from the first side.

2. A separation device according to the preceding claim,
**characterised in that**,
with respect to their cross-section perpendicularly to the flow direction, the flow channel and/or the inlet channels and/or the outlet channels are spatially formed so that, when fluids which are usable for immunomagnetic separation flow through the flow channel, a laminar flow or a flow with a Reynolds number R smaller than the critical Reynolds number R_{crit} can be generated therein and so that a first fluid flow can be formed in the flow channel on the side of the first magnet (6) and a second fluid flow which is separate from the first fluid flow except for diffusion processes can be formed in the flow channel on the side of the second magnet (7).

3. A separation device according to either one of the preceding claims,
**characterised by**
a first magnet (6) and a second magnet (7) for generating magnetic fields over at least part of the cross-section of the flow channel (5),
wherein two inlet channels (1, 2) lead into the intake region (E) for the supply of fluids into the flow channel (5) and wherein two outlet channels (3, 4) lead out of the discharge region (A) for the removal of fluids,
wherein the first magnet (6) is arranged downstream of the intake region (E) laterally outside the flow channel (5) or at least partly integrated into the wall (5a) of the flow channel (5) or in the flow channel inside the wall (5a),
wherein, downstream of the first magnet (6) and upstream of the discharge region (A), the second magnet (7) is arranged laterally outside the flow channel (5) or at least partly integrated into the wall (5a) of the flow channel (5) or in the flow channel inside the wall (5a), and
wherein the first and the second magnet are arranged on substantially opposing sides of the flow channel (5) outside or inside the flow channel or integrated into the wall (5a) thereof.

4. A separation device according to any one of the preceding claims,
**characterised in that**
the two inlet channels (1, 2) and the two outlet channels (3, 4) and preferably also the first and the second magnet are arranged substantially in one plane in the flow direction of the flow channel (5).

5. A separation device according to any one of the preceding claims,
**characterised in that**
at least one of the inlet channels (1, 2) and/or at least one of the outlet channels (3, 4) leads in or leads out substantially in the flow direction of the flow channel (5) or inclined at an angle α, where 0 < α < 180°, especially where 0 < α < 90°, especially where 0 < α < 45°.

6. A separation device according to any one of the preceding claims,
**characterised in that**
a respective one of the inlet channels and one of the outlet channels are arranged on the same side of the flow channel, seen perpendicularly to the flow direction of the flow channel.

7. A separation device according to any one of the preceding claims,
**characterised in that**
the first and/or the second magnet (6, 7) is a permanent magnet or an electromagnet.

8. A separation device according to the preceding claim,
**characterised in that**
the field strength and/or the field gradient of the electromagnet is temporally and/or locally variable or can be held constant.

9. A separation device according to any one of the preceding claims,
**characterised in that**
the flow channel is a microfluid channel, in particular with a cross-sectional area perpendicularly to the flow direction of more than 0.002 mm² and/or less than 1 mm², preferably more than 0.01 mm² and/or less than 0.06 mm².

10. A separation device according to any one of the preceding claims,
**characterised in that**
the flow channel is a tube with a substantially circular, elliptical, rectangular or square cross-section.

11. A separation device according to any one of the preceding claims,
**characterised by**
a reaction device (14, 15) which is arranged after the first magnet and before the second magnet in the flow direction and which lengthens the flow path.

12. A separation device according to the preceding claim,
**characterised in that**
the reaction device, which lengthens the flow path, comprises a reaction chamber (14) preferably arranged substantially on the side of the first magnet (6) and a flow breaker (15) arranged inside the flow channel (5).

13. A separation device according to the preceding claim,
**characterised in that**
the first fluid flow is deflectable into the reaction chamber (14) by the flow breaker (15).

14. An immunomagnetic separation device according to the preceding claim,
**characterised in that**
the reaction chamber (14) has a substantially Ω-shaped, semicircular or trapezoidal cross-section in a plane parallel to the flow direction of the flow channel and/or **in that** the flow breaker (15) substantially has a triangular or T-shaped cross-section in this plane.

15. A separation device according to any one of the three preceding claims,
**characterised in that**
the reaction chamber (14) is formed as a convexity of the wall (5a) of the flow channel (5) and is formed in one piece therewith or **in that** the reaction chamber (14) is formed as a separate component arranged in an opening in the wall (5a) of the flow channel (5).

16. A separation device according to any one of the preceding claims,
**characterised by**
a control device, in particular control electronics, for controlling the first and/or second magnet (6, 7) and/or a regulating device for regulating the flow speed in the flow channel (5) and/or in the inlet channels (1, 2) and/or outlet channels (3, 4).

17. A separation device according to any one of the preceding claims,
**characterised in that**
the separation device is implantable into a human or animal body or **in that** the separation device is usable outside the human or animal body, in particular as laboratory apparatus.

18. A separation device according to any one of the preceding claims,
**characterised in that**
a separating wall is arranged in at least part of the flow channel (5) in the flow direction of the fluid between the region of the inlet channels (1, 2) and the region of the outlet channels (3, 4) and prevents mixing of adjacent fluid flows fed in separately from one another.

19. A separation arrangement comprising
an immunomagnetic separation device and
a fluid, in particular a liquid, containing a plurality of immunomagnetic particles (8), in particular coupled to antibodies or coupled to antigen-specific tetramers,
**characterised in that**
the immunomagnetic separation device is formed according to any one of the preceding claims.

20. A separation arrangement according to the preceding claim,
**characterised in that**
the particles (8) have ferromagnetic and/or superparamagnetic properties and/or a substantially spherical shape.

21. A separation arrangement according to either one of the two preceding claims,
**characterised in that**
the flow channel (5) and/or the inlet channels (1, 2) and/or the outlet channels (3, 4) of the separation device have a formation such that, and/or the fluid or the liquid has a viscosity, density, temperature and average flow speed in the flow channel (5) such that a laminar flow or a flow with a Reynolds number R smaller than the critical Reynolds number R_{crit} exists in the flow channel.

22. A separation arrangement according to any one of the three preceding claims,
**characterised in that**
the flow rate of the fluid containing the particles (8) or of the liquid containing the particles through the flow channel is more than 0.1 µl/min and/or less than 2000 µl/min, in particular more than 1 µl/min and/or less than 200 µl/min.

23. A separation arrangement according to any one of the four preceding claims,
**characterised in that**
the average flow speed of the fluid containing the particles (8) or of the liquid containing the particles in the flow channel is more than 0.03 mm/s and/or less than 3000 mm/s, in particular more than 0.3 mm/s and/or less than 300 mm/s.

24. An isolation method for the isolation of a specific biological material (11) outside a human or animal body, in particular an antigen, from a first fluid (9), in particular a liquid, optionally containing further biological and/or other materials (12) in addition to this biological material (11) with the aid of a second fluid (10), in particular a liquid, containing a plurality of immunomagnetic particles (8), in particular coupled to the antibodies, tetramers and/or streptamers specific to this antigen,
wherein the first fluid (9) and simultaneously the second fluid (10) are fed into a flow channel (5) so that laminar flow conditions are formed in the flow channel (5) between the two fluid flows fed in, and the first fluid (9) flows in a first fluid flow and the second fluid (10) flows in a second, adjacent fluid flow through the flow channel,
wherein, with the aid of a first magnetic field, the immunomagnetic particles (8) are at least partly drawn out of the second fluid flow into the first fluid flow, where they are then left to bond to the specific biological material (11) for a bonding period corresponding to part of the time required to flow through the flow channel, before the immunomagnetic particles (8, 13), at least some of which may be bonded to the specific biological material, are at least partly drawn out of the first fluid flow into the second fluid flow with the aid of a second magnetic field,
and wherein the two fluid flows are discharged separately from the flow channel (5).

25. An isolation method according to the preceding claim,
**characterised in that**
an immunomagnetic separation device or an immunomagnetic separation arrangement according to any one of claims 1 to 23 is used.

26. An isolation method according to either one of the two preceding claims,
**characterised in that**
the field strength and/or the gradient strength of the first and/or second magnetic field is selected so that it is sufficient for the transfer of the immunomagnetic particles (8) from the second to the first fluid flow and for the transfer of the immunomagnetic particles (13), at least some of which are bonded to the specific biological material, from the first to the second fluid flow.

27. An isolation method according to any one of the three preceding claims,
**characterised in that**
the first and/or second magnetic field is generated in a pulsed manner or is sinusoidally modulated.

28. An immunomagnetic isolation method according to any one of the four preceding claims,
**characterised in that**
the bonding period is increased by lengthening the flow path of the first fluid flow through the flow channel.

29. Use of an immunomagnetic separation device and/or an immunomagnetic separation arrangement and/or an immunomagnetic isolation method according to any one of the preceding claims for medical diagnosis or treatment outside a human or animal body.

## Revendications

1. Dispositif de séparation comprenant :
un canal d'écoulement (5) avec une paroi (5a), une zone d'entrée (E) et un zone de sortie (A) ménagée en aval de celle-ci, et
au moins un aimant (6, 7) pour générer un champ magnétique sur au moins une partie de la section transversale du canal d'écoulement (5),
dans lequel deux canaux d'admission (1, 2) pour l'acheminement de fluides dans le canal d'écoulement (5) débouchent dans la zone d'entrée (E) et deux canaux d'évacuation (3, 4) pour le transport de fluides s'éloignent de la zone de sortie (A),
dans lequel un premier aimant (6) génère un premier champ magnétique en aval de la zone d'entrée (E),
dans lequel le premier aimant (6) ou un autre aimant (7) génère un second champ magnétique en aval du premier champ magnétique et en amont de la zone de sortie (A), le premier aimant et le second aimant étant sensiblement opposés en ce qui concerne leur sens d'écoulement ou leur polarité, et
dans lequel le canal d'écoulement et/ou les canaux d'admission et/ou les canaux d'évacuation est/sont aménagé(s) et/ou réalisé(s) de manière à pouvoir générer un écoulement laminaire ou un écoulement avec un nombre de Reynolds R inférieur au nombre de Reynolds critique R_{crit}, dans le canal d'écoulement lors de l'écoulement du fluide utilisable pour la séparation immunomagnétique, former un premier courant de liquide dans le canal d'écoulement sur un premier côté et former dans le canal d'écoulement, sur un second côté différent du premier côté, un second courant de liquide séparé du premier courant de liquide n'incluant pas des processus de diffusion.

2. Dispositif de séparation selon la revendication précédente,
**caractérisé en ce que**
l'on réalise le canal d'écoulement et/ou les canaux d'admission et/ou les canaux d'évacuation d'un point de vue spatial, en ce qui concerne leur section transversale, perpendiculairement au sens d'écoulement de manière à pouvoir générer un écoulement laminaire ou un écoulement avec un nombre de Reynolds R inférieur au nombre de Reynolds critique R_{crit} dans le canal d'écoulement lors de l'écoulement du fluide utilisable pour la séparation immunomagnétique et former dans le canal d'écoulement, du côté du premier aimant (6), un premier courant de liquide et former dans le canal d'écoulement, du côté du second aimant (7), un second courant de liquide, séparé du premier courant de n'incluant pas des processus de diffusion.

3. Dispositif de séparation selon l'une quelconque des revendications précédentes,
**caractérisé par**
un premier aimant (6) et un second aimant (7) destinés à générer des champs magnétiques sur au moins une partie de la section transversale du canal d'écoulement (5),
dans lequel deux canaux d'admission (1, 2) pour l'acheminement de fluides dans le canal d'écoulement (5) débouchent dans la zone d'entrée (E) et deux canaux d'évacuation (3, 4) pour le transport de fluides s'éloignent de la zone de sortie (A),
dans lequel le premier aimant (6) est aménagé dans le canal d'écoulement en aval de la zone d'entrée (E) latéralement en dehors du canal d'écoulement (5) ou du moins de manière partiellement intégrée à la paroi (5a) du canal d'écoulement (5) ou à l'intérieur de la paroi (5a),
dans lequel le second aimant (7) est aménagé, en aval du premier aimant (6) et en amont de la zone de sortie (A), dans le canal d'écoulement latéralement en dehors du canal d'écoulement (5) ou du moins de manière partiellement intégrée à la paroi (5a) du canal d'écoulement (5) ou à l'intérieur de la paroi (5a), et
dans lequel le premier aimant et le second aimant sont aménagés sur des côtés sensiblement opposés du canal d'écoulement (5) en dehors ou à l'intérieur du canal d'écoulement ou intégrés à sa paroi (5a).

4. Dispositif de séparation selon l'une quelconque des revendications précédentes,
**caractérisé en ce que**
les deux canaux d'admission (1, 2), les deux canaux d'évacuation (3, 4) et également, de préférence, les premier et second aimants sont aménagés sensiblement dans un plan dans le sens d'écoulement du canal d'écoulement (5).

5. Dispositif de séparation selon l'une quelconque des revendications précédentes,
**caractérisé en ce que**
au moins un des canaux d'admission (1, 2) et/ou au moins un des canaux d'évacuation (3, 4) débouche(nt) ou s'éloigne(nt), à cet égard de manière inclinée, sensiblement dans le sens d'écoulement du canal d'écoulement (5) ou sous un angle α avec 0 < α < 180°, en particulier avec 0 < α < 90°, plus particulièrement avec 0 < α < 45°.

6. Dispositif de séparation selon l'une quelconque des revendications précédentes,
**caractérisé en ce que**,
respectivement, un des canaux d'admission et un des canaux d'évacuation, tels que vus perpendiculairement au sens d'écoulement du canal d'écoulement, sont aménagés sur le même côté du canal d'écoulement.

7. Dispositif de séparation selon l'une quelconque des revendications précédentes,
**caractérisé en ce que**
le premier aimant et/ou le second aimant (6, 7) est/sont un aimant permanent ou un électroaimant.

8. Dispositif de séparation selon la revendication précédente,
**caractérisé en ce que**
l'on peut faire varier l'intensité de champ et/ou le gradient de champ de l'électroaimant en fonction du temps et/ou du l'emplacement ou **en ce que** l'on peut les maintenir constant.

9. Dispositif de séparation selon l'une quelconque des revendications précédentes,
**caractérisé en ce que**
le canal d'écoulement est un canal de microfluide, ayant en particulier une surface de section transversale perpendiculaire au sens d'écoulement supérieure à 0,002 mm² et/ou inférieure à 1 mm², de préférence supérieure à 0,01 mm² et/ou inférieure 0,06 mm².

10. Dispositif de séparation selon l'une quelconque des revendications précédentes,
**caractérisé en ce que**
le canal d'écoulement est un tube sensiblement circulaire, elliptique, rectangulaire ou carré en section transversale.

11. Dispositif de séparation selon l'une quelconque des revendications précédentes,
**caractérisé par**
un dispositif de réaction (14, 15) prolongeant le chemin d'écoulement qui est ménagé dans le sens d'écoulement après le premier aimant et avant le second aimant.

12. Dispositif de séparation selon la revendication précédente,
**caractérisé en ce que**
le dispositif de réaction prolongeant le chemin d'écoulement présente une chambre de réaction (14) ménagée de préférence sensiblement sur le côté du premier aimant (6) et un dispositif briseur d'écoulement (15) ménagé à l'intérieur du canal d'écoulement (5).

13. Dispositif de séparation selon la revendication précédente,
**caractérisé en ce que**
le premier courant de liquide peut être orienté dans la chambre de réaction (14) à l'aide du dispositif briseur d'écoulement (15).

14. Dispositif de séparation immunomagnétique selon la revendication précédente,
**caractérisé en ce que**
la chambre de réaction (14) présente, dans un plan parallèlement au sens d'écoulement du canal d'écoulement, une section transversale sensiblement en forme de Ω, semi-circulaire ou trapézoïdale et/ou **en ce que** le dispositif briseur d'écoulement (15) présente dans ce plan essentiellement une section transversale triangulaire ou en forme de T.

15. Dispositif de séparation selon l'une quelconque des trois revendications précédentes,
**caractérisé en ce que**
la chambre de réaction (14) se présente sous la forme d'un bombement de la paroi (5a) du canal d'écoulement (5) ou est réalisée d'un seul tenant avec celui-ci ou **en ce que** la chambre de réaction (14) se présente sous la forme d'un composant séparé ménagé à une ouverture de la paroi (5a) du canal d'écoulement (5).

16. Dispositif de séparation selon l'une quelconque des revendications précédentes,
**caractérisé par**
un dispositif de contrôle, en particulier un système électronique de contrôle pour commander le premier et/ou le second aimant (6, 7) et/ou un dispositif de réglage pour régler le débit dans le canal d'écoulement (5) et/ou dans les canaux d'admission (1, 2) et/ou dans les canaux d'évacuation (3, 4).

17. Dispositif de séparation selon l'une quelconque des revendications précédentes,
**caractérisé en ce que**
le dispositif de séparation est implantable dans le corps d'un homme ou d'un animal ou **en ce que** le dispositif de séparation est utilisable en dehors du corps d'un homme ou d'un animal, en particulier comme appareil de laboratoire.

18. Dispositif de séparation selon l'une quelconque des revendications précédentes,
**caractérisé en ce que**
l'on aménage dans le canal d'écoulement (5) dans le sens d'écoulement du fluide entre la zone des canaux d'admission (1, 2) et la zone des canaux d'évacuation (3, 4), du moins par zone, une paroi de séparation qui empêche un mélange des courants de fluide adjacents et introduits séparément l'un de l'autre.

19. Aménagement de séparation comprenant :
un dispositif de séparation immunomagnétique et
un fluide présentant une pluralité de particules (8) immunomagnétiques, en particulier couplées à des anticorps ou couplées à des tétramères spécifiques à un antigène, en particulier un liquide,
**caractérisé en ce que**
le dispositif de séparation immunomagnétique est réalisé selon l'une quelconque des revendications précédentes.

20. Aménagement de séparation selon la revendication précédente,
**caractérisé en ce que**
les particules (8) présentent des propriétés ferromagnétiques et/ou superparamagnétiques et/ou une forme sensiblement sphérique.

21. Aménagement de séparation selon l'une quelconque des deux revendications précédentes,
**caractérisé en ce que**
le canal d'écoulement (5) et/ou les canaux d'admission (1, 2) et/ou les canaux d'évacuation (3, 4) du dispositif de séparation sont réalisés et/ou **en ce que** le fluide et/ou le liquide présente une viscosité, une densité, une température et une vitesse d'écoulement moyenne dans le canal d'écoulement (5) de sorte qu'il y ait dans le canal d'écoulement un écoulement laminaire ou un écoulement avec un nombre de Reynolds R inférieur au nombre de Reynolds critique R_{crit}.

22. Aménagement de séparation selon l'une quelconque des trois revendications précédentes,
**caractérisé en ce que**
les débits d'écoulement du fluide présentant les particules (8) ou du liquide présentant les particules à travers le canal d'écoulement sont supérieures à 0,1 µl/min et/ou inférieures à 2000 µl/min, en particulier supérieures à 1 µl/min et/ou inférieures à 200 µl/min.

23. Aménagement de séparation selon l'une quelconque des quatre revendications précédentes,
**caractérisé en ce que**
la vitesse d'écoulement moyenne du fluide présentant les particules (8) ou du liquide présentant les particules dans le canal d'écoulement est supérieur à 0,03 mm/s et/ou inférieur à 3000 mm/s, en particulier supérieur à 0,3 mm/s et/ou inférieur à 300 mm/s.

24. Procédé d'isolement pour isoler un matériau biologique déterminé (11) en dehors du corps d'un homme ou d'un animal, en particulier un antigène, d'un premier fluide (9) présentant, outre ce matériau biologique (11), éventuellement d'autres matériaux biologiques et/ou d'autres matériaux (12), en particulier d'un liquide, à l'aide d'un second fluide (10) présentant une pluralité de particules (8) immunomagnétiques, en particulier couplées aux anticorps, tétramères et/ou streptamères spécifiques à cet antigène, en particulier d'un liquide,
dans lequel le premier fluide (9) et, en même temps, le second fluide (10) sont introduits dans un canal d'écoulement (5) de sorte qu'il se forme des rapports d'écoulement laminaire entre les deux courants de fluide introduits dans le canal d'écoulement (5) et que le premier fluide (9) s'écoule dans un premier courant de liquide et le second fluide (10) dans un second courant de liquide adjacent à travers le canal d'écoulement,
dans lequel les particules immunomagnétiques (8) passent du moins partiellement du second courant de liquide au premier courant de liquide à l'aide d'un premier champ magnétique, puis y sont laissées pour la liaison avec le matériau biologique déterminé (11) pendant une certaine période de temps de liaison correspondant à une partie de la période de temps nécessaire à la traversée du canal d'écoulement, avant que les particules immunomagnétiques (8, 13) éventuellement liées, du moins partiellement, au matériau biologique déterminé passent du moins partiellement du premier courant de liquide au second courant de liquide à l'aide d'un second champ magnétique, et
dans lequel les deux courants de liquide sont évacués séparément du canal d'écoulement (5).

25. Procédé d'isolement selon la revendication précédente,
**caractérisé en ce que**
l'on utilise un dispositif de séparation immunomagnétique ou un aménagement de séparation immunomagnétique selon l'une quelconque des revendications 1 à 23.

26. Procédé d'isolement selon l'une quelconque des deux revendications précédentes,
**caractérisé en ce**
l'on choisit l'intensité de champ et/ou l'intensité de gradient du premier et/ou du second champ magnétique de sorte qu'elle(s) soit/soient juste suffisantes pour le passage des particules immunomagnétiques (8) du second courant de liquide au premier courant de liquide ou pour le passage des particules immunomagnétiques (13) liées du moins partiellement au matériau biologique déterminé du premier courant de liquide au second courant de liquide.

27. Procédé d'isolement selon l'une quelconque des trois revendications précédentes,
**caractérisé en ce que**
le premier et/ou le second champ magnétique est/sont généré(s) de manière pulsée ou modulé(s) de manière sinusoïdale.

28. Procédé d'isolement immunomagnétique selon l'une quelconque des quatre revendications précédentes,
**caractérisé en ce**
la période de temps de liaison est augmentée en prolongeant le chemin d'écoulement du premier courant de liquide à travers le canal d'écoulement.

29. Utilisation d'un dispositif de séparation immunomagnétique et/ou d'un aménagement de séparation immunomagnétique et/ou d'un procédé d'isolement immunomagnétique selon l'une quelconque des revendications précédentes à des fins de diagnostic médical ou de thérapie en dehors du corps d'un homme ou d'un animal.
